# EUROPÄISCHE PATENTSCHRIFT

(11) **EP 4 606 351 B1**
(45) Veröffentlichungstag und Bekanntmachung des Hinweises auf die Patenterteilung: **26.08.2026**
(21) Anmeldenummer: 25151530.0
(22) Anmeldetag: 13.01.2025
(51) Int. Cl.: A61F 2/18

(54) **EINSEITIGES IMPLANTAT ZUR SPREIZUNG EINES NASENFLÜGELS**
SINGLE FACE IMPLANT FOR SPREADING A NASAL WING
IMPLANT MONOFACE DESTINÉ À ÉLARGIR UNE AILE NASALE

(30) Priorität: 20.02.2024 DE 102024104626
(43) Veröffentlichungstag der Anmeldung: 27.08.2025
(73) Patentinhaber: Heinz Kurz GmbH, 72144 Dusslingen (DE)
(72) Erfinder: SANDER, Alexander, 79822 Titisee-Neustadt (DE); STIEGELE, Thomas, 72585 Riederich (DE); MERTENS, Matthias, 24568 Kaltenkirchen (DE); À WENGEN, Daniel Felix, 4103 Bottmingen (CH)
(74) Vertreter: Kohler Schmid Möbus Patentanwälte

(56) Entgegenhaltungen:
- EP-A1- 1 475 056
- DE-B3- 102006 023 058
- DE-B3- 102022 120 193
- US-A1- 2014 039 619
- US-A1- 2021 077 292
- US-B2- 9 949 823

## Beschreibung

Die Erfindung betrifft ein rhinologisches Implantat zur Spreizung mindestens eines Nasenflügels, welches in der menschlichen Nase befestigbar ist, im implantierten Zustand auf mindestens einer Innenfläche eines Nasenflügels vollflächig anliegt und aus einem mit einer Vielzahl von durchgängigen Perforationen versehenen Zuschnitt aus dünnem Blech aufgebaut ist, wobei der zunächst flache Zuschnitt vor der Implantation in eine aus der Blechebene heraus gekrümmte Raumform aufgebogen ist, welche der natürlichen Biegung der Innenseite der weichen Nasenseitenwand folgt; wobei das Implantat im implantierten Zustand auf lediglich einer Innenfläche eines einzigen der beiden Nasenflügel vollflächig anliegt; wobei das Implantat einen Dreiecksförmigen Grundkörper aufweist, bei welchem von einer Dreiecksspitze ausgehend zwei Katheten-Kanten unter einem Dreieckswinkel φ gegeneinander angeordnet sind, jeweils von der Dreiecksspitze wegführen und beiderseits jeweils in Richtung auf eine der Dreiecksspitze gegenüberliegende Hypotenusen-Kante verlaufen; und wobei die Basisspitzen des dreiecksförmigen Grundkörpers, wo jeweils eine Katheten-Kante auf die der Dreiecksspitze gegenüberliegende Hypotenusen-Kante trifft, verkürzt beziehungsweise abgehackt ausgebildet sind, sodass das der Dreiecksspitze abgewandte Ende der jeweiligen Katheten-Kante über eine weitere Kantenlinie mit einem Ende der Hypotenusen-Kante verbunden ist.

Ein derartiges rhinologisches Implantat zur Spreizung von Nasenflügeln geht hervor aus dem generischen Stand der Technik US 9 949 823 B2 (= Referenz [1]).

Die DE 20 2008 010 203 U1 (= Referenz [2]) beschreibt ein rhinologisches Implantat mit rechteckigem Grundkörper.

Ein ähnliches Implantat ist in US 2021/0 077 292 A1 (= Referenz [3]) beschrieben, allerdings ohne explizite Erwähnung, dass der zunächst flache Zuschnitt vor der Implantation in eine aus der Blechebene heraus gekrümmte Raumform aufgebogen ist.

### Hintergrund der Erfindung

Generell sind Implantate zur Spreizung der Nasenflügel sowie deren Handhabung sehr ausführlich beschrieben beispielsweise auf folgenden Internetseiten (eingesehen am 30.01.2024):
Referenz [4a] (Prof. Dr. á Wengen)
   https://breatheimplant.com/de/
Referenz [4b] (die Anmelderin Firma Kurz)
   https://www.kurzmed.com/de/produkte/rhinologie/breathe-implant-awengen.html
Referenz [4c] (Firma Bess)
   https://bess.eu/rhinologie/breathe-implant-awengen

Die Einpflanzung eines Implantats, d. h. eines in der Regel körperfremden Gewebsstücks oder Stoffes, in den menschlichen Körper ist in der Medizintechnik ein lange bekanntes Verfahren, das in vielen Variationen zur Behebung funktioneller Störungen verschiedener Körperteile und/oder psychischer Beeinträchtigungen vorgenommen wird.

Eine Spreizung der Nasenflügel kann beispielsweise bei einer Verengung der Nasenklappe oder bei einem Kollaps der Weichteile der Nasenflügel indiziert sein.

Im Folgenden sollen zunächst die wesentlichen Unterschiede zwischen einem Eingriff zur Spreizung der Nasenflügel und der bekannten Columella-Strut-Technik erläutert werden:
Die Spreizung der Nasenflügel zielt ausschließlich darauf ab, den Naseninnenraum dauerhaft zu vergrößern und damit die Luftzufuhr über die Nase zu optimieren. Es handelt sich also um rein funktionelle Chirurgie. Hierzu gibt es eine Reihe von chirurgischen Techniken, die über Nähte und Transplantate zum Ziel führen sollen. Unterschiedliche und im Wesentlichen von der Erfahrung und dem Geschick des Operateurs abhängige Verfahren kommen hierfür in Betracht. Disartikulationstechniken bis hin zu gezielten Knorpelinzisionen können mehr oder weniger eine Rolle spielen.

Im Gegensatz dazu zielt die Columella-Strut-Technik darauf ab, die Nasenspitze aus kosmetischen Gründen heraus zu erhöhen. Dabei wird ein Steg zwischen die beiden Enden der beiden Flügelknorpel geschoben, um die Spitze der Nase zu erhöhen. Das Transplantat/ Implantat wird in eine Tasche zwischen den medialen Schenkeln der Flügelknorpel über die Spina nasalis anterior aufgestellt und zwischen den medialen Schenkeln mit durchgreifenden Nähten befestigt.

Dies ist beispielsweise beschrieben in der US 2012/0078367 A1 (= Referenz [5]). Hier wird von einem Implantat gesprochen, das biologisch abgebaut wird und somit nicht für den Langzeitverbleib vorgesehen ist. Die Zielregion ist das untere Drittel der Nase, hauptsächlich die Region um die Nasenspitze. Zwar ist hier auch die Rede von einer Anbindung des Implantates an das Septum, allerdings nicht zur Begradigung desselben, sondern zur Verlängerung, falls durch äußere Einwirkung oder aus kosmetischer Sicht das Septum verkürzt ist. Die gebogenen Teile des beschriebenen Implantats sind in ihrer Längenausdehnung höchstens etwa 20mm lang, d.h. sie reichen, wenn man sie auf die Basis der Spina Nasalis setzt und die beiden Schenkel der Flügelknorpel aufnimmt, gerade noch ans Ende des Septums. Bereits der Sitz dieses Implantats im implantierten Zustand wäre für eine Nasenflügel-Spreizung völlig ungeeignet.

Das Implantat nach Referenz [5] ist daher keinesfalls für eine Spreizung der Nasenflügel vorgesehen, und ist auch nicht beiderseits des Septums der menschlichen Nase auf der jeweiligen Außenfläche des Septums in der linken beziehungsweise rechten Seite der Nasenhöhle befestigbar. Vielmehr handelt es sich bei dem Implantat gemäß Referenz [5] um ein "Columella Strut", nicht jedoch um eine Vorrichtung entsprechend der vorliegenden Erfindung zur Spreizung der Nasenflügel. Ein Hilfsmittel für eine derartige Spreizung thematisiert und erwähnt Referenz [5] überhaupt nicht.

Auch in der WO 2008/153263 A1 (= Referenz [6]) werden ausschließlich Techniken beschrieben, die zum Ziel haben, die Nasenspitze anzuheben. So ist dort zwar vom Septum die Rede. Beschrieben wird etwa die Technik, dass das Implantat an der oberen Grenze des Septum-Knorpels angebracht werden muss. Allerdings geschieht dies auch hier wieder lediglich zur Stabilisierung des Nasenspitzenimplantats.

Die bekannten Implantations-Systeme nach Referenz [5] und nach Referenz [6] versuchen also, das Ende des Septums zur Stabilisierung oder zur Verlängerung zu verwenden. Keines der beiden Systeme zielt jedoch darauf ab oder wäre geeignet dazu, einen oder beide Nasenflügel dauerhaft vom Septum weg abzuspreizen.

CN 21 538 4901 U (= Referenz [7]) offenbart eine Nasenkorrekturstütze für die plastische Chirurgie, die einen Columella-nasi-Stützabschnitt, einen Nasenseptum-Verlängerungsabschnitt, Blättchen des Columella-nasi-Stützabschnitts, Blättchen des Nasenseptum-Verlängerungs-Abschnitts, eine Nasenspitzecke und eine Columella-nasi umfasst mit einer Klemmnut am Stützende, einer Klemmnut am Kopfende der Nasenscheidewandverlängerung und einer U-förmigen Nut.

US 11 241 306 B2 (= Referenz [8]) beschreibt Nasenimplantate mit einem planaren Profil, welches teilweise offene Räume aufweist. Ein solches Nasenimplantat kann kompressibel entlang einer oder mehrerer Dimensionen sein, wie etwa der Breite oder der Länge des planaren Profils. Jedenfalls ist ein solches Implantat nicht zur Spreizung der Nasenflügel geeignet und bedient daher ein ganz anderes, eigenständiges Versorgungsfeld.

Eine einfache und häufig angewandte Methode zur Spreizung der Nasenflügel besteht darin, ein Nasenstützpflaster zu verwenden, das insbesondere auch Hochleistungssportler zur Verbesserung der Nasenatmung einsetzen. Dieses Vorgehen ist jedoch zur dauerhaften Anwendung ungeeignet. Bei regelmäßiger Verwendung der Nasenpflaster können durch den Klebstoff Hautprobleme entstehen. Außerdem beeinträchtigt ein Nasenpflaster das äußere Erscheinungsbild eines Menschen.

Zur dauerhaften Spreizung der Nasenflügel sind daher auch operative Methoden bekannt, bei denen zur Stabilisierung der seitlichen Nasenweichteile Knorpel eingesetzt wird. Die Ergebnisse sind jedoch optisch und funktional nicht immer befriedigend. Außerdem bringt dieses Vorgehen noch andere Nachteile mit sich. Da vorzugsweise körpereigener Knorpel zur Stabilisierung verwendet wird, muss die Entnahme des Knorpels, vorzugsweise aus dem Ohr oder aus der Nasenscheidewand des Patienten, diesem Schritt vorangehen, was einen zeitlichen Aufwand und ein zusätzliches Risiko für den Patienten bedeutet. Oft zeigt sich, dass das eingesetzte Teil zu wenig Eigenspannung besitzt, um den kaudalen Luftraum der Nase in einer befriedigenden Weise offen zu halten. Das Verfahren kann sich sogar als kontraproduktiv erweisen, weil die eingesetzten Teile die Weichteile nach innen drücken und zu einer weiteren Verengung der Nase führen können.

Auch metallische Implantate werden zur Spreizung der Nasenflügel eingesetzt. Dazu wird operativ eine Öffnung im Bereich des Flügelrandes der Nase eingebracht, durch welche das Implantat eingesetzt und am Dreiecksknorpel befestigt wird. Metallische Implantate sind wesentlich fester und elastischer als Knorpel und besitzen genügend Eigenspannung, um den nasalen Luftraum in einer dauerhaften und befriedigenden Weise offen zu halten.

Nun weist der Dreiecksknorpel der menschlichen Nase bekanntlich einen relativ flachen oder allenfalls sehr leicht gekrümmten Plateaubereich auf. Nachteilig bei vielen bekannten Implantaten ist, dass sie um den Nasenrücken herum jeweils so stark gekrümmt sind, dass im implantierten Zustand im Bereich dieses Plateaus des Dreiecksknorpels ein Hohlraum zwischen Dreiecksknorpel und dem Implantat entsteht, der zu einem ungünstigen Abheilverhalten nach der Implantation und möglicherweise zu unkontrolliert wuchernden Gewebeansammlungen führen kann. Des Weiteren sind viele bekannte Implantate geometrisch so gestaltet, dass sie nicht optimal an den Flanken des Dreiecksknorpels im Bereich der Nasenflügel anliegen.

Ein weiteres dachförmiges Implantat zur Spreizung der Nasenflügel, das im implantierten Zustand wie auch die Implantate gemäß DE 10 2006 023 058 B3 ≈ EP 1 857 078 B1 (= Referenz [9]) oder aus DE 203 07 058 U1 ≈ DE 20 2004 021 075 U1 ≈ EP 1 475 056 B1 (= Referenz [10]) über den Rücken des Septums verläuft, ist in US 6 322 590 B1 (= Referenz [11]) beschrieben. Dieses Implantat ist ebenfalls aus einem geraden ebenen Metallstreifen. Der Metallstreifen weist an seinen beiden Enden Perforationen in Form von runden Löchern auf und wird aus der Ebene in eine räumlich V-förmige Dachform, allerdings mit "runder Spitze" des räumlichen V gebogen.

Implantate nach der Referenz [9], bei welchen der ebene Streifen ebenfalls aus der Ebene in eine räumlich V-förmige Dachform gebogen wird, jedoch in der Ebene bereits eine winkelförmige oder trapezförmige Kontur aufweist, ermöglichen geometrisch günstigere Implantations-Anordnungen in der Nase und stellen eine Verbesserung gegenüber dem rhinologischen Implantat gemäß Referenz [11] dar:
In Referenz [9] wird vorgeschlagen, dass ein Rückenabschnitt des Implantats oberhalb des Nasenrückens flach oder nur sehr leicht gegen den Plateaubereich des Dreiecksknorpels mit einem Spreizungswinkel ω>160° abgewinkelt oder tonnenförmig mit einem Krümmungsradius r>4cm, vorzugsweise r>10cm, um den Plateaubereich des Dreiecksknorpels herum gekrümmt ist, und dass zwei Seitenabschnitte des Implantats beiderseits der Nasenflügel im Wesentlichen parallel zum jeweiligen Nasenflügel unter einem Winkel φ von jeweils mehr als 50° gegen den flachen Rückenabschnitt nach unten abgekantet verlaufen. Damit wird ohne größeren Fertigungsaufwand eine besonders gute geometrische Anpassung des Implantats an die (normale) Ausgestaltung des menschlichen Dreiecksknorpels erreicht. Insbesondere entstehen nach der Operation keine Hohlräume zwischen dem Implantat und dem Dreiecksknorpel, weder im Bereich des Plateaus des Dreiecksknorpels noch an dessen Flanken. Vielmehr liegt das Implantat über seine gesamte dem Dreiecksknorpel zugewandte Oberfläche eng an diesem an, was auch einen besonders guten mechanischen Halt des Implantats am Dreiecksknorpel bewirkt.

### Aufgabe der Erfindung

Der vorliegenden Erfindung liegt demgegenüber die Aufgabe zugrunde, mit unaufwändigen technischen Mitteln ein rhinologisches Implantat der gattungsbildenden Art mit den eingangs definierten Merkmalen dahingehend zu modifizieren, dass die Vorrichtung nicht über den Nasenrücken-seitigen Teil des Septums gesetzt und auf beiden Seiten der Nasenscheidewand angebracht werden muss, sondern eine einseitige Anwendung ermöglicht wird. Außerdem soll der Fertigungsaufwand wesentlich verringert und ein erheblicher Anteil an Material und Gewicht eingespart werden. Im implantierten Zustand der Vorrichtung soll ein möglichst dauerhaft sicherer Sitz des Implantats erzielt werden. Schließlich soll sich das Implantat durch seine Formgebung besonders gut und optimal flächendeckend auf die Innenseite des Nasenflügels einpassen lassen.

### Kurze Beschreibung der Erfindung

Diese relativ komplexe Aufgabe wird auf überraschend einfach und kostengünstig zu realisierende Weise dadurch gelöst, dass der dreiecksförmige Grundkörper an den beiden Katheten-Kanten durch zwei jeweils von der Dreiecksspitze weg über die Hypotenusen-Kante hinausgreifende Seitenabschnitte verlängert ist, wobei die Außen-Kanten der beiden Seitenabschnitte jeweils in Flucht mit ihrer jeweiligen Katheten-Kante verlaufen.

Der Zuschnitt für derartige Implantate ist in der Herstellung nicht wesentlich komplizierter und die hinausgreifenden Seitenabschnitte bewirken eine Vergrößerung der Anlagefläche und verbessern den Sitz im implantierten Zustand.

Dadurch entsteht eine Art kompakte "Bumerang"-Form, die sich besonders gut und optimal flächendeckend auf die Innenseite des Nasenflügels einpassen lässt.

Wie auch schon die generischen rhinologischen Implantate dient das erfindungsgemäße Implantat insbesondere zur Stabilisierung, Stützung und Erweiterung der weichen Nasenseitenwand im Bereich zwischen dem unteren Ende des Dreieckknorpels der menschlichen Nase, des knöchernen Naseneingangs «Apertura piriformis», des Nasenrückens und des Naseneingangs einseitig (oder bei mehrfacher Anwendung des Implantats sogar beidseitig). Das erfindungsgemäße Implantat trägt damit dazu bei, das Ansaugen der Nasenseitenwand zu reduzieren oder aufzuheben und dadurch die Nasenatmung zu erleichtern und mithin zu verbessern.

Die neuartige Grundidee der Erfindung ist dabei, ein lediglich einseitig einsetzbares Implantat vorzustellen, welches nur auf einer Seite des Septums an der Innenwand des entsprechenden Nasenflügels aufgebracht wird. Dadurch wird die Implantations-OP wesentlich vereinfacht, weil zum Einsetzen des Implantats eben nur noch auf einer Seite des Septums an einer Innenfläche eines Nasenflügels operiert werden muss. Insbesondere muss hierzu nicht mehr die Nasenschleimhaut vom Knorpel der Scheidewand abgehoben werden. Vielmehr wird das leicht gekrümmte, im Wesentlichen starre erfindungsgemäße Implantat sofort auf seine gewünschte Position an der Innenwand eines Nasenflügels eingeschoben.

Zwar können theoretisch auch zwei Implantate auf beiden Seiten des Septums an den Innenseiten beider Nasenflügel eingesetzt werden, um etwa eine besonders hohe mechanische Stabilität zu erreichen oder um asymmetrische Deformationen der Nasen-Scheidewand feinfühliger und besser korrigieren zu können. Auch in solchen Fällen wird aber immerhin durch das Fehlen eines Nasenrücken-Abschnitts am erfindungsgemäßen Implantat die Operation deutlich unaufwändiger und damit weniger belastend für den Patienten.

Durch den erfindungsgemäßen speziellen geometrischen Aufbau des Implantats mit einem im Wesentlichen dreiecksförmigen Grundkörper wird eine relativ große Anlagefläche an der Innenseite des Nasenflügels geschaffen, die einen sicheren, stabilen Sitz des Implantats auch bei Langzeit-Anwendung sowie bei den dann zu erwartenden zahlreichen mechanischen Bewegungen der Patienten-Nase (etwa beim Schnäuzen) garantieren kann.

Die Materialeinsparung bei der Herstellung des erfindungsgemäßen Implantats gegenüber dem oben diskutierten generischen Stand der Technik ist ebenfalls erheblich: Aufgrund des Weglassens des bisher stets obligatorischen Nasenrücken-Abschnitts sowie des gegenüberliegenden weiteren Seitenteils werden 60-70% geringere Materialmengen benötigt, was natürlich auch zu einer großen Gewichtseinsparung beim erfindungsgemäßen Implantat führt.

Schließlich wird auch das Fertigungsverfahren ganz wesentlich vereinfacht:
Das Implantat wird nach dem Ausstanzen des ursprünglichen flachen, ungebogenen Zuschnitt in eine lediglich sehr leicht gekrümmte, der natürlichen Biegung der Innenseite der weichen Nasenseitenwand folgende Raumform aufgebogen. Damit lässt sich das erfindungsgemäße Implantat besser in die vorhandene Struktur der Patienten-Nase einbringen. Ein sehr starkes Aufbiegen (wie etwa bei den generischen Implantaten gemäß Referenz [9] oder [10] jeweils zwingend erforderlich, schon wegen der Ausformung des dort immer vorhandenen Nasenrückenteils relativ zu den beiden dort ebenfalls immer vorhandenen Seitenabschnitten in eine Dachform) entfällt nun vollständig, was wiederum zu einer deutlichen Arbeitsersparnis bei der Herstellung, aber nachfolgend auch beim Einsetzen des erfindungsgemäßen Implantats während der Implantation beiträgt.

Damit wird trotz des gegenüber dem Stand der Technik deutlich geringeren Fertigungsaufwands eine besonders gute geometrische Anpassung des Implantats an die aktuelle, möglicherweise auch stark deformierte Nasensituation des jeweiligen Patienten und eine besonders gute Anlage des Implantats an einer der Innenflächen der Nasenflügel erreicht. Insbesondere können nach der Operation keine Hohlräume zwischen dem Implantat und dem Septum entstehen (wie beim Stand der Technik durchaus möglich und teilweise sogar unausweichlich). Vielmehr liegt das im Wesentlichen flache, lediglich ganz leicht gekrümmte erfindungsgemäße Implantat nach der Operation über seine gesamte dem Nasenflügel zugewandte Oberfläche eng an diesem an, was auch einen besonders guten Sitz und mechanischen Halt des Implantats in der Nase bewirkt.

Durch vorkonfektionierte erfindungsgemäße Implantate kann der Eingriff besonders gut reproduzierbar, standardisiert und planbar durchgeführt werden. Wenn eine gewisse Anzahl verschiedener Größen und auch unterschiedlicher Formen des erfindungsgemäßen Implantats vorgehalten wird, kann der Operateur besonders genau und feinfühlig auf die individuellen geometrischen Verhältnisse in der jeweiligen Patienten-Nase optimal eingehen. Eine Hauptaufgabe dabei ist immer, die weiche Nasenseitenwand dauerhaft zu stabilisieren.

### Bevorzugte Ausführungsformen der Erfindung

In der Praxis bewähren sich Ausführungsformen des erfindungsgemäßen rhinologischen Implantats, bei welchen zumindest eine der beiden Katheten-Kanten und/oder die der Dreiecksspitze gegenüberliegende Hypotenusen-Kante nicht geradlinig linear, sondern einer gekrümmten Kurve folgend verläuft.

Damit lassen sich besonders "weiche" Außenkonturen des Implantats realisieren und eine optimale Anpassung an die tatsächlichen geometrischen Gegebenheiten auf der Innenseite des Nasenflügels bewirken.

Ganz besonders bevorzugt sind Ausführungsformen des erfindungsgemäßen Implantats, welche sich dadurch auszeichnen, dass die Dreiecksspitze sowie vorzugsweise auch alle weiteren, zumindest die außenliegenden, Eckstrukturen des Implantats abgerundet ausgeführt sind.

Damit lässt sich einerseits während der Implantation ein Hängenbleiben oder gar Aufspießen sicher verhindern, andererseits wird dadurch auch im implantierten Zustand bei etwaigen post-operativen Bewegungen des Implantats relativ zu seiner Umgebung eine Verletzungsgefahr ausgeschlossen oder zumindest minimiert. Auch das Einwachsen des Implantats in das menschliche Gewebe wird dadurch erleichtert und die Bedeckung durch die Weichteile der Nasenseitenwand verbessert.

In einer Klasse von besonders bevorzugten Ausführungsformen ist das Implantat symmetrisch zu einer von der Dreiecksspitze rechtwinklig auf die Hypotenusen-Kante verlaufenden (gedachten) Höhen-Achse aufgebaut, die das Implantat lateral in zwei spiegelbildliche Hälften aufteilt.

Diese symmetrische Formgebung ist fertigungstechnisch besonders einfach und präzise zu realisieren.

Alternativ dazu zeichnet sich eine weitere Klasse von Ausführungsformen der Erfindung dadurch aus, dass das Implantat asymmetrisch aufgebaut ist, wobei eine erste Katheten-Kante eine größere Längenausdehnung von der Dreiecksspitze des dreiecksförmigen Grundkörpers weg aufweist als die zweite Katheten-Kante.

Auf diese Weise kann in einem Sortiment mit unterschiedlichen Formen und Größen auf individuelle Besonderheiten und ungewöhnliche Deformationen der jeweiligen Patienten-Nase, die sich mit einer symmetrischen Form des Implantats nicht optimal behandeln ließen, besondere Rücksicht genommen werden.

Vorteilhafte Varianten des erfindungsgemäßen Implantats zeichnen sich daher auch dadurch aus, dass in den dreiecksförmigen Grundkörper ein oder mehrere Spikes eingearbeitet sind.

Letztere verkrallen sich bei der Implantation im Nasenflügel und sorgen so für einen exzellenten und äußerst dauerhaften Halt des Implantats. Diese Art der Befestigung kann freilich auch mit anderen der oben beschriebenen Befestigungsarten kombiniert werden, um einen besonders gesicherten Sitz des Implantats am Nasenflügel zu garantieren.

Wie bereits oben beschrieben, weist das erfindungsgemäße Implantat eine Vielzahl von Perforationen auf. Damit wird einerseits die Masse des Implantats reduziert und mithin dessen Gewicht verringert, andererseits der Anteil von körperfremdem Material, das durch das Implantat in den menschlichen Körper eingebracht wird, so weit wie möglich minimiert. Außerdem fördern die Perforationen das Verwachsen des Implantats mit dem Gewebe. Insbesondere sind die Perforationen wichtig, um die Versorgung der Weichteile auf beiden Seiten des Implantats sicherzustellen, um ein Durchwachsen von Gewebe und damit eine sichere und dauerhafte Fixierung des Implantats im Gewebe zu ermöglichen, um ein Annähen des Implantats an den Knochen der Apertura piriformis und eine Fixierung an den äußersten Teil des Flügelknorpels zu ermöglichen.

Die Perforationen sind vorzugsweise sowohl am dreiecksförmigen Grundkörper als auch an den beiden Seitenabschnitten des Implantats vorhanden und können zum Beispiel als kreisrunde Löcher, als Langlöcher oder auch als anders geformte Öffnungen ausgebildet sein.

Weiter dienen diese Perforationen auch einer sicheren Fixierung durch Annähen des Implantats mittels einer OP-Naht. Damit kann zudem sichergestellt werden, dass das betroffene Gewebe ausreichend mit Nährstoffen versorgt wird.

Die Herstellung einer dauer-haltbaren Befestigung des Implantats durch Aufnähen ist allerdings in der Regel zeitintensiv und manchmal auch aufgrund der räumlichen Verhältnisse etwas kompliziert.

Bei weiteren vorteilhaften Ausführungsformen der Erfindung können die Perforationen des Implantats zusammen einen größeren Flächeninhalt aufweisen als die die Perforationen umgebenden, insbesondere stegförmig ausgebildeten, festen Teilabschnitte des Implantats.

Damit lassen sich die Masse und mithin das Gewicht des erfindungsgemäßen Implantats bei dennoch hoher mechanischer Stabilität deutlich minimieren. Auch wird hierdurch eine ausreichende Versorgung des Gewebes mit Nährstoffen sichergestellt.

Vorzugsweise kann bei Varianten der Erfindung zumindest ein Teil der Perforationen polyederförmig oder wabenförmig ausgebildet sein. Dies ermöglicht eine hohe Stabilität auch bei geringem Materialeinsatz.

Zumindest ein Teil der Perforationen kann aber auch schlitzförmig, vorzugsweise durch untereinander parallel verlaufende längliche Schlitze, ausgebildet sein.

Damit wird unter anderem die Nahtführung für den implantierenden Chirurgen erleichtert.

Besonders bevorzugt sind Ausführungsformen des erfindungsgemäßen Implantats, bei welchen sämtliche Kanten des Implantats, zumindest alle außenliegenden Kanten, abgerundet ausgeführt sind.

Damit wird eine mögliche Verletzungsgefahr minimiert, das Einwachsen in das menschliche Gewebe wird erheblich erleichtert und die Bedeckung durch die Weichteile etwa einer Knorpelhaut sowie der Nasenschleimhaut wird merklich verbessert.

In der Praxis bewähren sich Ausführungsformen der Erfindung, bei welchen die Dicke des flachen Zuschnitts für das Implantat zwischen 0,2mm und 1,2mm, vorzugsweise etwa 0,8mm, beträgt.

Das erfindungsgemäße Implantat kann bis maximal die Ausdehnung der gesamten Innenfläche eines menschlichen Nasenflügels einnehmen und weist eine größte Längenausdehnung in der Zuschnitt-Ebene zwischen 2mm und 7mm, vorzugsweise etwa 5mm auf.

Für die allermeisten denkbaren Anwendungsfälle des erfindungsgemäßen Implantats eignen sich Ausführungsformen, bei denen der Dreieckswinkel φ an der Dreiecksspitze des dreiecksförmigen Grundkörpers zwischen 60° und 160°, vorzugsweise zwischen 100° und 120°, beträgt.

Diese Formgebung dient der Verstärkung des Implantats und vermindert damit das Eintreten einer Verbiegung durch äußere Kräfte im Sinne eines Knotenblechs.

Für einige Anwendungen kann es vorteilhaft sein, wenn das Implantat ganz oder teilweise aus Metall gefertigt ist. Als Material für chirurgische und orthopädische Implantate, die dauerhaft im Körper verbleiben sollen, sind Metalle und deren Legierungen prädestiniert, weil sie neben einer sehr guten Biokompatibilität hohe Dauerfestigkeit und Elastizität aufweisen. Trotz einer relativ geringen Dichte besitzen Implantate aus solchen Materialien wie reines Titan oder Titanverbindungen exzellente mechanische Eigenschaften mit einer langen Lebensdauer. Ebenfalls eine gute Eignung für die genannten Zwecke besitzen nicht-magnetisierbare Metalle beziehungsweise Legierungen, mit denen auch MRT-Sicherheitsaspekte berücksichtigt werden können.

Bevorzugt sind Ausführungsformen, bei welchen das Implantat aus einem Werkstoff mit Memory-Effekt und/oder superelastischen Eigenschaften, vorzugsweise aus Nitinol, aufgebaut ist, so dass beispielsweise durch geeignete thermische Behandlung des Implantats optimale Federeigenschaften relativ zum Septum eingebracht werden können.

Für andere Anwendungen kann es vorteilhaft sein, wenn das Implantat ganz oder teilweise aus Kunststoffblech gefertigt ist, insbesondere aus Polymer-Material, vorzugsweise aus einem Material, welches eine ähnliche Flexibilität wie die menschlichen Nasenflügel aufweist, aber über genügend Steifigkeiten verfügt, um eine dauerhafte Abspreizung des entsprechenden Nasenflügels vom Septum weg zu garantieren.

Zusätzlich zur guten Biokompatibilität des verwendeten Materials selbst kann das Implantat auch einen besonderen körperverträglichen und/oder keimabweisenden Überzug aufweisen.

Zur Erzielung einer fein abgestimmten Form des Implantats kann dieses zweckmäßigerweise mittels 3D-Drucktechnik und/oder mittels Lasertechnik hergestellt sein.

Entsprechend den individuellen Anforderungen kann das erfindungsgemäße Implantat aber auch in spanender Technik, insbesondere mittels Frästechnik und/oder mittels Schleiftechnik, vorzugsweise Gleitschleifen, Magnetgleitschleifen oder SatellitenFliehkraftschleifen, und/oder mittels Poliertechnik, vorzugsweise Elektropolieren, oder in Ätztechnik hergestellt werden.

Bei weiteren vorteilhaften Ausführungsformen der Erfindung schließlich ist das Implantat im Spritzguss nach dem Micro-Injection-Moulding-(=MIM) Verfahren gefertigt, welches beispielsweise aus der WO 00/06327 A2 (= Referenz [12]) an sich bekannt ist. Damit kann eine äußerst preisgünstige Herstellung auch sehr großer Stückzahlen bei gleichbleibender Qualität erreicht werden, während die herkömmlichen Implantate in der Regel wie etwa Schmuckwaren handangefertigt werden und daher einerseits relativ teuer in der Herstellung sind, andererseits in der Maßgenauigkeit individuell variieren können.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen.

### Detaillierte Beschreibung der Erfindung anhand der Zeichnung

In der schematischen Zeichnung sind Ausführungsbeispiele der Erfindung dargestellt, welche in der nachfolgenden Beschreibung näher erläutert werden.

Es zeigen:
- Fig. 1: eine schematische Draufsicht in stark vergrößerter Darstellung senkrecht von oben auf die Zuschnitts-Ebene einer symmetrischen Ausführungsform eines Implantats mit exakt spiegelbildlich zueinander ausgebildeten Seitenabschnitten in großer L-Größe;
- Fig. 2: eine Draufsicht auf den Zuschnitt für eine Ausführungsform des erfindungsgemäßen Implantats mit symmetrisch ausgreifenden Seitenabschnitten;
- Fig. 3: eine Ausführungsform mit asymmetrischen Seitenabschnitten;
- Fig. 4: eine asymmetrische Ausführungsform mit einem ausgreifenden Seitenabschnitt auf der einen Seite und einer abgehackten Basisspitze auf der anderen Seite;
- Fig. 5a: eine schematische Draufsicht senkrecht von oben auf die Zuschnitts-Ebene einer asymmetrischen Ausführungsform eines Implantats mit beiderseits abgehackten Basisspitzen sowie mit einer gekrümmten Katheten-Kante und einer gekrümmten Hypotenusen-Kante;
- Fig. 5b: die Ausführungsform von Fig. 5a in gekrümmter räumlicher Darstellung;
- Fig. 6a: eine ähnliche Ausführungsform eines Implantats wie in Fig. 5a als räumliche Draufsicht auf den -bereits leicht gekrümmten- Zuschnitt;
- Fig. 6b: die Ausführungsform von Fig. 6a mit Blickrichtung in der Ebene des gekrümmten Zuschnitts;
- Fig. 6c: die Ausführungsform von Fig. 6a in räumlicher Darstellung schräg von der Seite;
- Fig. 7a: eine schematische Draufsicht senkrecht von oben auf die Zuschnitts-Ebene einer asymmetrischen Ausführungsform ähnlich wie in den Fign. 5 und 6 mit Bemaßung; und
- Fig. 7b: die Ausführungsform von Fig. 7a mit Blickrichtung in der Ebene des gekrümmten Zuschnitts mit Bemaßung.

Das in sämtlichen Figuren der Zeichnung dargestellte rhinologische **Implantat 10; 20; 30; 40; 50; 60** zur Spreizung mindestens eines Nasenflügels ist in der menschlichen Nase befestigbar. Im implantierten Zustand liegt es auf mindestens einer Innenfläche eines Nasenflügels vollflächig an und ist aus einem mit einer Vielzahl von durchgängigen **Perforationen 15** versehenen Zuschnitt aus dünnem Blech aufgebaut.

Die Perforationen 15 können -wie in den Figuren der Zeichnung gezeigtpolyederförmig oder wabenförmig, aber bei nicht eigens dargestellten Ausführungsformen auch als kreisrunde Löcher oder auch als Langlöcher ausgebildet sein. Sie helfen, einerseits das Gewicht des Implantats zu verringern und andererseits den Anteil des körperfremden Materials im Körper eines Patienten so weit wie möglich zu reduzieren. Außerdem fördern die Perforationen 15 das Verwachsen des Implantats 10; 20; 30; 40; 50; 60 mit dem umgebenden Gewebe.

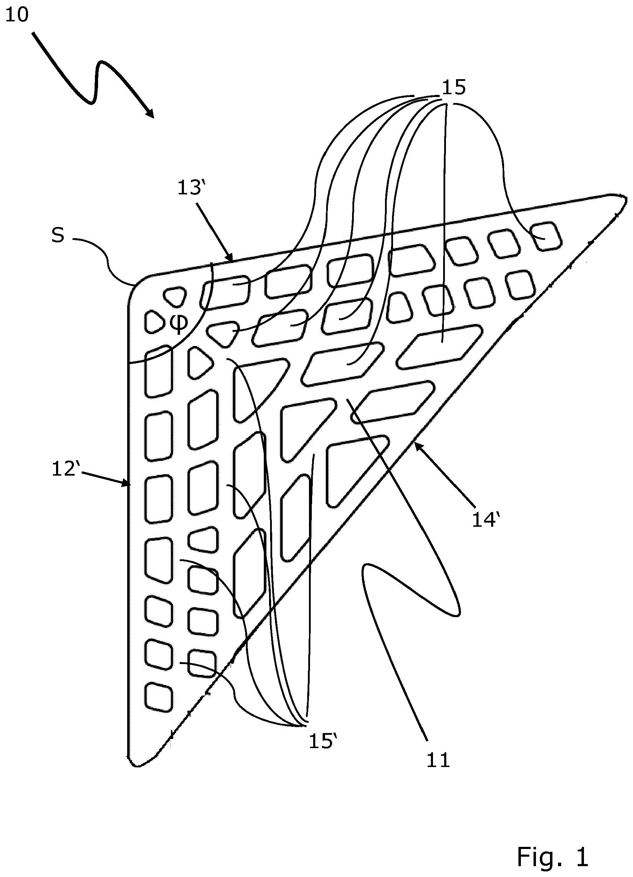

Die Perforationen 15 weisen bei den in den **Figuren 1 bis 4** **sowie 6** dargestellten Ausführungsformen in Summe einen größeren Flächeninhalt auf als die die Perforationen 15 umgebenden, stegförmig ausgebildeten, **festen Teilabschnitte 15'** des Implantats 10; 20; 30; 40; 60. Vorzugsweise ist zumindest ein Teil der Perforationen 15 polyederförmig oder wabenförmig ausgebildet. Möglich sind aber auch andere Formen, wie etwa längliche oder kreisrunde Löcher.

Das Implantat 10; 20; 30; 40; 50; 60 wird operativ in die menschliche Nase eingebracht und mittels einer Naht befestigt. Hierbei werden mehrere Einzelnähte durch die Perforationen 15 angelegt und fixiert.

Das Implantat 10; 20; 30; 40; 50; 60 zeichnet sich unter anderem dadurch aus, dass der zunächst flache Zuschnitt vor der Implantation in eine aus der Blechebene heraus gekrümmte Raumform aufgebogen ist, welche der natürlichen Biegung der Innenseite der weichen Nasenseitenwand folgt.

Im Gegensatz zu den bisher bekannten, das Septum übergreifenden beidseitigen Implantaten liegt das Implantat 10; 20; 30; 40; 50; 60 im implantierten Zustand auf lediglich einer Innenfläche eines einzigen der beiden Nasenflügel vollflächig an.

Bei -in der Zeichnung nicht eigens dargestellten- Anwendungen können allerdings auch erfindungsgemäße Implantate auf beiden Nasenflügeln angebracht werden. In diesen Fällen werden die beiden verwendeten Implantate in der Regel zueinander spiegelbildlich ausgebildet sein. Freilich sind auch Anwendungsfälle denkbar, wo aufgrund einer individuellen Asymmetrie oder sonstige Abweichungen in der Geometrie der beiden Nasenflügel eines Patienten auch erfindungsgemäße Implantate von unterschiedlicher Größe in den beiden Nasenhälften zur Anwendung kommen können. Gegenüber den bekannten beidseitigen Implantaten bietet das erfindungsgemäß stets nur in einer Nasenhälfte anbringbare Implantat jedenfalls dem behandelnden Otologen eine wesentlich höhere Flexibilität, um optimal auf die Besonderheiten des jeweiligen Einzelfalls einzugehen.

Das Implantat 10; 20; 30; 40; 50; 60 weist einen in etwa **dreiecksförmigen Grundkörper 11; 21; 31; 41; 51; 61** auf, bei welchem von einer **Dreiecksspitze S** ausgehend zwei **Katheten-Kanten 12',13'; 22',23'; 32',33'; 42',43'; 52',53'; 62',63'** unter einem **Dreieckswinkel φ** gegeneinander angeordnet sind, jeweils von der Dreiecksspitze S wegführen und beiderseits jeweils in Richtung auf eine der Dreiecksspitze S gegenüberliegende **Hypotenusen-Kante 14'; 24'; 34'; 44'; 54'; 64'** verlaufen.

Bei sämtlichen in der Zeichnung dargestellten Ausführungsformen sind die Dreiecksspitze S sowie auch alle weiteren, zumindest die außenliegenden, Ecken des Implantats 10; 20; 30; 40; 50; 60 abgerundet ausgeführt.

Die Blechdicke des flachen Zuschnitts für das Implantat 10; 20; 30; 40; 50; 60 beträgt in der Regel zwischen 0,2mm und 1,2mm, vorzugsweise etwa 0,8mm, und die maximale Längsausdehnung zwischen 2mm und 7mm, vorzugsweise etwa 5mm.

Der Dreieckswinkel φ an der Dreiecksspitze S des dreiecksförmigen Grundkörpers 11; 21; 31; 41; 51; 61 kann zwischen 60° und 160° betragen, vorzugsweise zwischen 100° und 120°. In den gezeigten Ausführungsformen liegt er bei ungefähr 100°.

Das Implantat 10; 20; 30; 40; 50; 60 kann ganz oder teilweise aus Metall, insbesondere aus Titan, vorzugsweise aus Rein-Titan oder einer Titanlegierung, oder aus Edelstahl und/oder aus einem Werkstoff mit Memory-Effekt und/oder superelastischen Eigenschaften, vorzugsweise aus Nitinol, gefertigt sein. Vorzugsweise wird es einen körperverträglichen, antibakteriellen Überzug aufweisen.

Das Implantat 10; 20; 30; 40; 50; 60 kann aber auch ganz oder teilweise aus Kunststoffblech gefertigt sein, insbesondere aus Polymer-Material, vorzugsweise aus einem Material, welches eine ähnliche Flexibilität wie die menschlichen Nasenflügel aufweist, aber über genügend Steifigkeiten verfügt, um eine dauerhafte und stabile Abspreizung eines Nasenflügels vom Septum zu bewirken.

Das Implantat 10; 20; 30; 40; 50; 60 kann mittels 3D-Drucktechnik und/oder mittels Lasertechnik hergestellt werden. Möglich ist auch, das Implantat 10; 20; 30; 40; 50; 60 in spanender Technik, insbesondere mittels Frästechnik und/oder mittels Schleiftechnik, vorzugsweise Gleitschleifen, Magnetgleitschleifen oder Satellitenfliehkraftschleifen, und/oder mittels Poliertechnik, vorzugsweise Elektropolieren, oder in Ätztechnik herzustellen und zu bearbeiten. Außerdem kann das Implantat 10; 20; 30; 40; 50; 60 beispielsweise auch im Spritzguss nach dem Micro-Injection-Moulding-(=MIM)-Verfahren gefertigt werden.

**Fig. 1** zeigt eine geometrisch besonders einfache Ausführungsform eines Implantats 10, bei welcher das gesamte Implantat allein aus dem dreiecksförmigen Grundkörper 11 besteht.

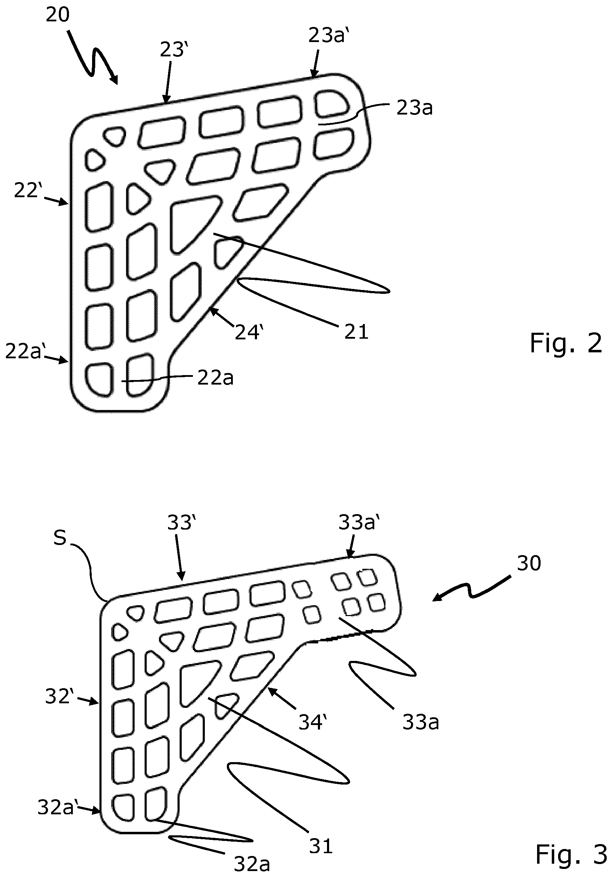

Bei anderen Ausführungsformen der Erfindung können aber auch Teile des Grundkörpers verkürzt oder verlängert werden:
So zeigen die **Figuren 2 bis 4** Ausführungsformen des erfindungsgemäßen Implantats 20; 30; 40, bei welchen der dreiecksförmige Grundkörper 21; 31; 41 an einer oder beiden Katheten-Kanten 22',23'; 32',33'; 43' durch zwei jeweils von der Dreiecksspitze S weg über die Hypotenusen-Kante 24'; 34'; 44' **hinausgreifende Seitenabschnitte 22a,23a; 32a,33a; 43a** verlängert ist, wobei die **Außen-Kanten 22a',23a'; 32a',33a'; 43a'** der beiden Seitenabschnitte 22a,23a; 32a,33a; 43a jeweils in Flucht mit ihrer jeweiligen Katheten-Kante 22',23'; 32',33'; 43' verlaufen.

Bei den in den **Figuren 1** **und** **2** dargestellten Ausführungsformen ist das Implantat 10; 20 *symmetrisch* zu einer von der Dreiecksspitze S rechtwinklig auf die Hypotenusen-Kante 14'; 24' verlaufenden (gedachten) Höhen-Achse aufgebaut, die das Implantat 10; 20 lateral in zwei spiegelbildliche Hälften aufteilt.

Ausführungsbeispiele für einen *asymmetrischen* Aufbau des Implantats 30; 40; 50; 60 sind in den **Figuren 3 bis 6c** gezeigt. Dort weist die erste Katheten-Kante 32'; 42'; 52'; 62' eine größere Längenausdehnung von der Dreiecksspitze S des dreiecksförmigen Grundkörpers 31; 41; 51; 61 weg auf als die zweite Katheten-Kante 33'; 43'; 53'; 63'.

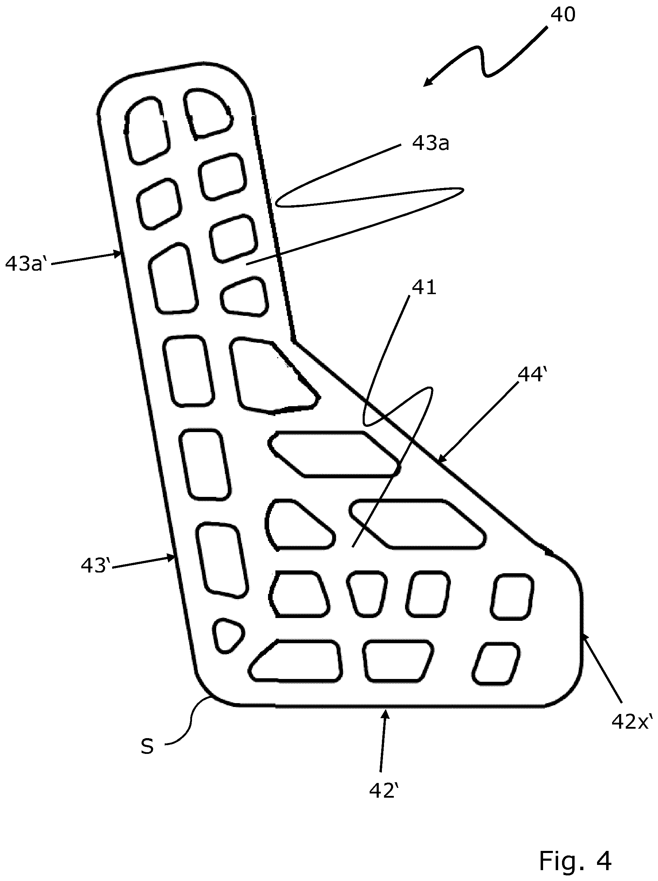

Wie aus den Ausführungsformen der **Figuren 4 bis 6c** zu erkennen ist, kann zumindest eine, gegebenenfalls auch beide der Basisspitzen des dreiecksförmigen Grundkörpers 41; 51; 61, wo jeweils eine Katheten-Kante 42'; 52',53'; 62',63' auf die der Dreiecksspitze S gegenüber liegende Hypotenusen-Kante 44'; 54'; 64' trifft, verkürzt beziehungsweise abgehackt ausgebildet sein, sodass das der Dreiecksspitze S abgewandte Ende der jeweiligen Katheten-Kante 42'; 52',53'; 62',63' über eine **weitere Kantenlinie 42x'; 52x',53x'; 62x',63x'** mit einem Ende der Hypotenusen-Kante 44'; 54'; **64'** verbunden ist.

Des Weiteren müssen die Kantenlinien des Implantats nicht notwendigerweise linear verlaufen:

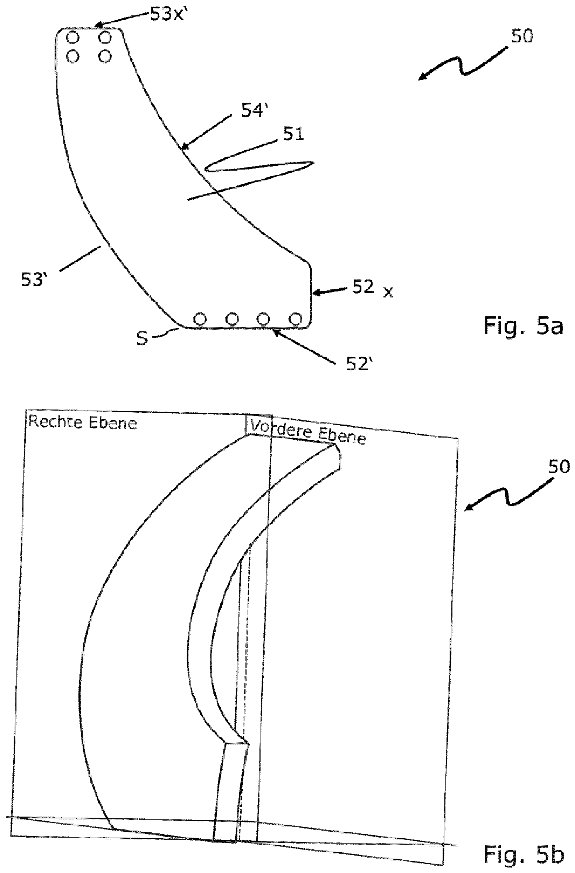

Die **Figuren 5a bis 6c** zeigen Ausführungsformen, bei welchen zumindest eine der beiden Katheten-Kanten 53'; 63' und/oder die der Dreiecksspitze S gegenüberliegende Hypotenusen-Kante 54'; **64'** nicht geradlinig linear, sondern *einer gekrümmten Kurve folgend* verläuft.

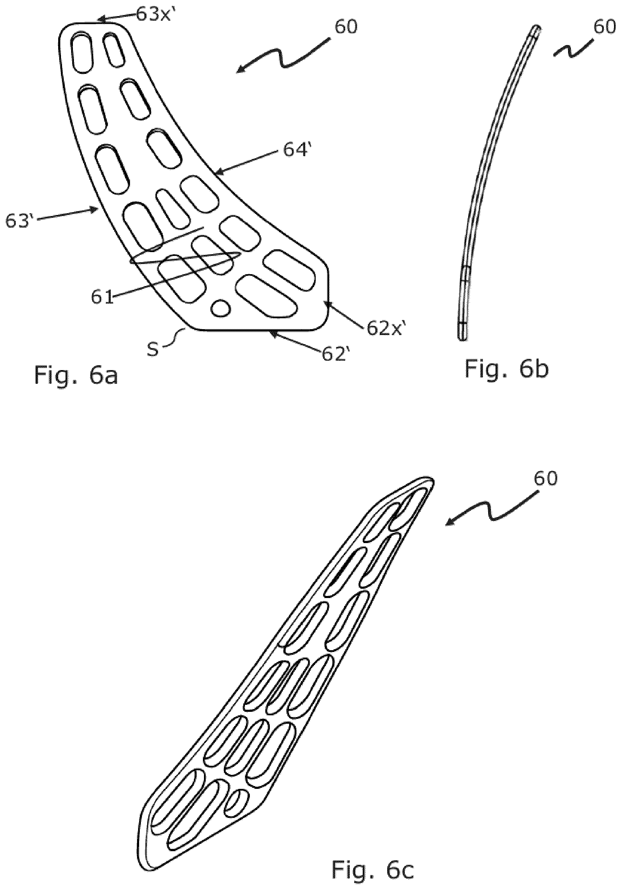

Wie etwa bei der in **Fig. 6c** räumlich dargestellten Ausführungsform zu erkennen ist, können beim Implantat 10; 20; 30; 40; 50; 60 auch einige oder sämtliche Kanten 12',13',14'; 22',23',24', 22a',23a'; 32',33',34',32a',33a'; 42',43',44',42x',43a'; 52',53',54', 52x',53x'; 62',63',64',62x',63x', insbesondere zumindest alle außenliegenden Kanten, in der Blechebene des Zuschnitts abgerundet ausgeführt sein.

Nicht eigens in der Zeichnung dargestellt sind Ausführungsformen der Erfindung, bei welchen in den dreiecksförmigen Grundkörper 11; 21; 31; 41; 51; 61 und gegebenenfalls auch in die Seitenabschnitte 22a,23a; 32a,33a; 43a ein oder mehrere Spikes eingearbeitet sind.

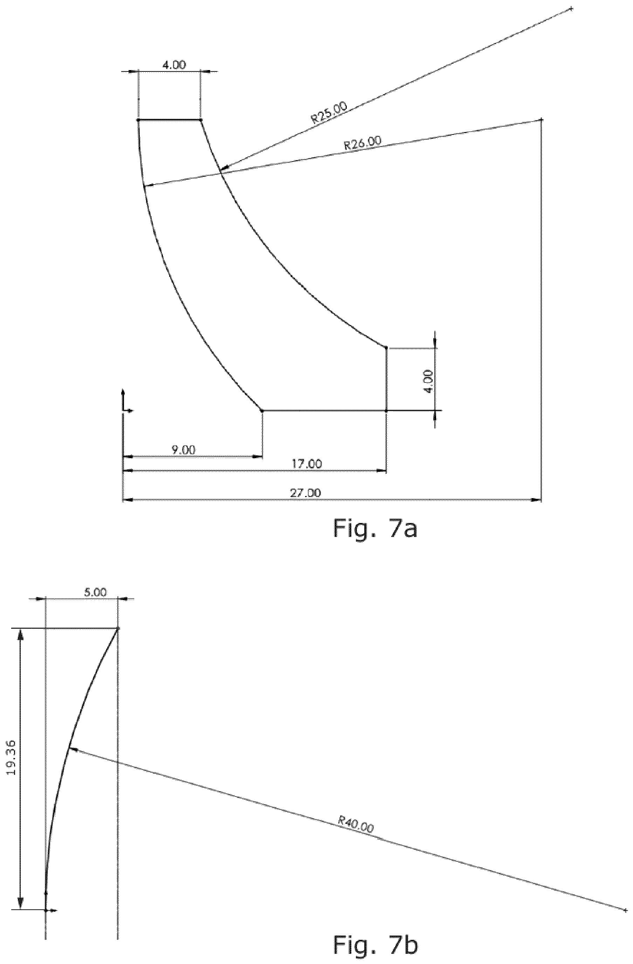

Typische Auslegungen der Bemaßung des Zuschnitts für die Bumerangförmigen Ausführungsformen des Implantats 50; 60 sind in den **Figuren 7a und 7b** gezeigt. Insbesondere erkennt man in Fig. 7a die jeweiligen Krümmungsradien der nicht-linear verlaufenden Hypotenusen- und Katheten-Kante und in Fig. 7b den Radius der geringen Krümmung aus der Zuschnitts-Ebene.

Hauptzweck des erfindungsgemäßen Implantats ist eine dauerhafte Abspreizung eines Nasenflügels von der Nasenscheidewand. Indikationen können zum Beispiel Deviationen in Folge von Traumata, kongenitaler Deviation usw. oder einfach nur Atembeschwerden des Patienten in Folge eines zu nahe am Septum lokalisierten Nasenflügels und der dadurch verursachten Strömungsverengung der Atemluftströmung sein.

### Bezugszeichenliste:

- 10; 20; 30; 40; 50; 60: rhinologisches Implantat
- 11; 21; 31; 41; 51; 61: dreiecksförmiger Grundkörper
- 12',13'; 22',23'; 32',33'; 42',43'; 52',53'; 62',63': Katheten-Kanten
- 14'; 24'; 34'; 44'; 54'; 64': Hypotenusen-Kante
- 22a,23a; 32a,33a; 43a: hinausgreifende Seitenabschnitte
- 22a',23a'; 32a',33a'; 43a': Außen-Kanten der Seitenabschnitte
- 42x'; 52x',53x'; 62x',63x': weitere Kantenlinie
- 15: Perforationen
- 15': feste Teilabschnitte
- S: Dreiecksspitze
- φ: Dreieckswinkel

### Referenzliste:

Für die Beurteilung der Patentfähigkeit in Betracht gezogene Publikationen:
[1] US 9 949 823 B2
[2] DE 20 2008 010 203 U1
[3] US 2021/0 077 292 A1
[4a] Internetseite von Prof. Dr. á Wengen vom 30.01.2024
   https://breatheimplant.com/de/
[4b] Internetseite der Firma Kurz vom 30.01.2024
   https://www.kurzmed.com/de/produkte/rhinologie/breathe-implant-awengen.html
[4c] Internetseite der Firma Bess vom 30.01.2024
   https://bess.eu/rhinologie/breathe-implant-awengen
[5] US 2012/0078367 A1
[6] WO 2008/153263 A1
[7] CN 21 538 4901 U
[8] US 11 241 306 B2
[9] DE 10 2006 023 058 B3 ≈ EP 1 857 078 B1
[10] DE 203 07 058 U1 ≈ DE 20 2004 021 075 U1 ≈ EP 1 475 056 B1
[11] US 6 322 590 B1
[12] WO 00/06327 A2

## Patentansprüche

1. Implantat (10; 20; 30; 40; 50; 60) zur Spreizung mindestens eines Nasenflügels, welches in der menschlichen Nase befestigbar ist, im implantierten Zustand auf mindestens einer Innenfläche eines Nasenflügels vollflächig anliegt und aus einem mit einer Vielzahl von durchgängigen Perforationen (15) versehenen Zuschnitt aus dünnem Blech aufgebaut ist; wobei der zunächst flache Zuschnitt vor der Implantation in eine aus der Blechebene heraus gekrümmte Raumform aufgebogen ist, welche der natürlichen Biegung der Innenseite der weichen Nasenseitenwand folgt; wobei das Implantat (10; 20; 30; 40; 50; 60) im implantierten Zustand auf lediglich einer Innenfläche eines einzigen der beiden Nasenflügel vollflächig anliegt; wobei das Implantat (10; 20; 30; 40; 50; 60) einen dreiecksförmigen Grundkörper (11; 21; 31; 41; 51; 61) aufweist, bei welchem von einer Dreiecksspitze (S) ausgehend zwei Katheten-Kanten (12',13'; 22',23'; 32',33'; 42',43'; 52',53'; 62',63') unter einem Dreieckswinkel (φ) gegeneinander angeordnet sind, jeweils von der Dreiecksspitze (S) wegführen und beiderseits jeweils in Richtung auf eine der Dreiecksspitze (S) gegenüberliegende Hypotenusen-Kante (14'; 24'; 34'; 44'; 54'; 64') verlaufen; und wobei die Basisspitzen des dreiecksförmigen Grundkörpers (41; 51; 61), wo jeweils eine Katheten-Kante (42'; 52',53'; 62',63') auf die der Dreiecksspitze (S) gegenüberliegende Hypotenusen-Kante (44'; 54'; 64') trifft, verkürzt beziehungsweise abgehackt ausgebildet sind, sodass das der Dreiecksspitze (S) abgewandte Ende der jeweiligen Katheten-Kante (42'; 52',53'; 62',63') über eine weitere Kantenlinie (42x'; 52x',53x'; 62x',63x') mit einem Ende der Hypotenusen-Kante (44'; 54'; 64') verbunden ist,
**dadurch gekennzeichnet,**
**dass** der dreiecksförmige Grundkörper (21; 31; 41) an einer oder beiden Katheten-Kanten (22',23'; 32',33'; 43') durch zwei jeweils von der Dreiecksspitze (S) weg über die Hypotenusen-Kante (24'; 34'; 44') hinausgreifende Seitenabschnitte (22a,23a; 32a,33a; 43a) verlängert ist, wobei die Außen-Kanten (22a',23a'; 32a',33a'; 43a') der beiden Seitenabschnitte (22a,23a; 32a,33a; 43a) jeweils in Flucht mit ihrer jeweiligen Katheten-Kante (22',23'; 32',33'; 43') verlaufen.

2. Implantat nach Anspruch 1, **dadurch gekennzeichnet, dass** zumindest eine der beiden Katheten-Kanten (53'; 63') und/oder die der Dreiecksspitze (S) gegenüberliegende Hypotenusen-Kante (54'; 64') nicht geradlinig linear, sondern einer gekrümmten Kurve folgend verläuft.

3. Implantat nach einem der vorhergehenden Ansprüche, **dadurch gekennzeichnet, dass** die Dreiecksspitze (S) sowie vorzugsweise auch alle weiteren, zumindest die außenliegenden, Ecken des Implantats (10; 20; 30; 40; 50; 60) abgerundet ausgeführt sind.

4. Implantat nach einem der Ansprüche 1 bis 3, **dadurch gekennzeichnet, dass** das Implantat (10; 20) symmetrisch zu einer von der Dreiecksspitze (S) rechtwinklig auf die Hypotenusen-Kante (14'; 24') verlaufenden Höhen-Achse, die das Implantat (10; 20) lateral in zwei spiegelbildliche Hälften aufteilt, aufgebaut ist.

5. Implantat nach einem der Ansprüche 1 bis 3, **dadurch gekennzeichnet, dass** das Implantat (30; 40; 50; 60) asymmetrisch aufgebaut ist, wobei eine erste Katheten-Kante (32'; 42'; 52'; 62') eine größere Längenausdehnung von der Dreiecksspitze (S) des dreiecksförmigen Grundkörpers (31; 41; 51; 61) weg aufweist als die zweite Katheten-Kante (33'; 43'; 53'; 63').

6. Implantat nach einem der vorhergehenden Ansprüche, **dadurch gekennzeichnet, dass** in den dreiecksförmigen Grundkörper (11; 21; 31; 41; 51; 61) ein oder mehrere Spikes eingearbeitet sind.

7. Implantat nach einem der vorhergehenden Ansprüche, **dadurch gekennzeichnet, dass** zumindest ein Teil der Perforationen (15) als kreisrunde Löcher, als Langlöcher, als längliche Schlitze, polyederförmig oder wabenförmig ausgebildet ist

8. Implantat nach einem der vorhergehenden Ansprüche, **dadurch gekennzeichnet, dass** die Perforationen (15) zusammen einen größeren Flächeninhalt aufweisen als die die Perforationen (15) umgebenden, insbesondere stegförmig ausgebildeten, festen Teilabschnitte (15') des Implantats (10; 20; 30; 40; 50; 60).

9. Implantat nach einem der vorhergehenden Ansprüche, **dadurch gekennzeichnet, dass** sämtliche Kanten (12',13',14'; 22',23',24', 22a',23a'; 32',33',34',32a',33a'; 42',43',44',42x',43a'; 52',53',54', 52x',53x'; 62',63',64',62x',63x') des Implantats (10; 20; 30; 40; 50; 60), zumindest alle außenliegenden Kanten, in der Blechebene des Zuschnitts abgerundet ausgeführt sind.

10. Implantat nach einem der vorhergehenden Ansprüche, **dadurch gekennzeichnet, dass** die Blechdicke des flachen Zuschnitts für das Implantat (10; 20; 30; 40; 50; 60) zwischen 0,2mm und 1,2mm, vorzugsweise etwa 0,8mm, und die maximale Längsausdehnung zwischen 2mm und 7mm, vorzugsweise etwa 5mm beträgt.

11. Implantat nach einem der vorhergehenden Ansprüche, **dadurch gekennzeichnet, dass** der Dreieckswinkel (φ) an der Dreiecksspitze (S) des dreiecksförmigen Grundkörpers (11; 21; 31; 41; 51; 61) zwischen 60° und 160°, vorzugsweise zwischen 100° und 120°, beträgt.

12. Implantat nach einem der vorhergehenden Ansprüche, **dadurch gekennzeichnet, dass** das Implantat (10; 20; 30; 40; 50; 60) einen körperverträglichen Überzug aufweist.

13. Implantat nach einem der Ansprüche 1 bis 12, **dadurch gekennzeichnet, dass** das Implantat (10; 20; 30; 40; 50; 60) aus Metallblech, insbesondere aus reinem Titan oder einer Titanlegierung, oder aus einem Werkstoff mit Memory-Effekt und/oder superelastischen Eigenschaften, vorzugsweise aus Nitinol, aufgebaut ist.

14. Implantat nach einem der Ansprüche 1 bis 12, **dadurch gekennzeichnet, dass** das Implantat (10; 20; 30; 40; 50; 60) ganz oder teilweise aus Kunststoffblech gefertigt ist, insbesondere aus Polymer-Material, vorzugsweise aus einem Material, welches eine ähnliche Flexibilität wie die menschlichen Nasenflügel aufweist, aber über genügend Steifigkeit verfügt, um eine dauerhafte und stabile Abspreizung eines Nasenflügels vom Septum zu bewirken.

## Claims

1. Implant (10; 20; 30; 40; 50; 60) for spreading at least one nostril, which can be secured within the human nose, lies fully faced across the surface of at least one inner surface of a nostril when implanted, and is constructed from a blank of thin sheet metal provided with a plurality of through perforations (15); wherein the initially flat blank is bent prior to implantation into a three-dimensional shape curved out of the plane of the sheet, which follows the natural curvature of the inner surface of the soft lateral nasal wall; wherein the implant (10; 20; 30; 40; 50; 60) rests in full contact with only one inner surface of a single one of the two nasal wings when implanted; wherein the implant (10; 20; 30; 40; 50; 60) comprises a triangular base body (11; 21; 31; 41; 51; 61), in which, starting from a triangular vertex (S), two cathetus edges (12', 13'; 22', 23'; 32', 33'; 42', 43'; 52', 53'; 62', 63') are arranged at a triangular angle (φ) to one another, each extending away from the triangular vertex (S) and, on either side, each extending towards a hypotenuse edge (14'; 24'; 34'; 44'; 54'; 64'); and wherein the base vertices of the triangular base body (41; 51; 61), where in each case a leg edge (42'; 52', 53'; 62', 63') meets the hypotenuse edge (44'; 54'; 64') opposite the triangular vertex (S), are shortened or truncated, so that the end of the respective leg edge (42'; 52', 53'; 62', 63') is connected to one end of the hypotenuse edge (44'; 54'; 64') via a further edge line (42x'; 52x', 53x'; 62x', 63x'),
**characterised in**
**that** the triangular base body (21; 31; 41) is connected at one or both cathetus edges (22', 23'; 32', 33'; 43') by two side sections (22a, 23a; 32a, 33a; 43a), each extending away from the triangular vertex (S) beyond the hypotenuse edge (24'; 34'; 44'), whereby the outer edges (22a', 23a'; 32a', 33a'; 43a') of the two side sections (22a, 23a; 32a, 33a; 43a) each lie in line with their respective cathetus edge (22', 23'; 32', 33'; 43').

2. An implant according to claim 1, **characterised in that** at least one of the two catheter edges (53'; 63') and/or the hypotenuse edge (54'; 64') opposite the triangular vertex (S) of the triangle does not run in a straight line but follows a curved path.

3. An implant according to any one of the preceding claims, **characterised in that** the triangular vertex (S) of the triangle and, preferably, all other corners of the implant (10; 20; 30; 40; 50; 60) - at least the outer ones - are rounded.

4. An implant according to any one of claims 1 to 3, **characterised in that** the implant (10; 20) is constructed symmetrically about a height axis extending at right angles from the triangular vertex (S) to the hypotenuse edge (14'; 24'), which divides the implant (10; 20) laterally into two mirror-image halves.

5. An implant according to any one of claims 1 to 3, **characterised in that** the implant (30; 40; 50; 60) is constructed asymmetrically, wherein a first catheter edge (32'; 42'; 52'; 62') extends further away from the triangular vertex (S) of the triangular base body (31; 41; 51; 61) than the second side (33'; 43'; 53'; 63').

6. An implant according to one of the preceding claims, **characterised in that** one or more spikes are incorporated into the triangular base body (11; 21; 31; 41; 51; 61).

7. An implant according to any one of the preceding claims, **characterised in that** at least some of the perforations (15) are formed as circular holes, as elongated holes, as elongated slits, in a polyhedral shape or in a honeycomb shape

8. An implant according to any one of the preceding claims, **characterised in that** the perforations (15) together have a larger surface area than the solid sections (15') of the implant (10; 20; 30; 40; 50; 60) surrounding the perforations (15), in particular those formed as webs.

9. An implant according to one of the preceding claims, **characterised in that** all edges (12', 13', 14'; 22', 23', 24', 22a', 23a'; 32', 33', 34', 32a', 33a'; 42', 43', 44', 42x', 43a'; 52', 53', 54', 52x', 53x'; 62', 63', 64', 62x', 63x') of the implant (10; 20; 30; 40; 50; 60), at least all the outer edges, are rounded in the sheet metal plane of the blank.

10. An implant according to any one of the preceding claims, **characterised in that** the sheet thickness of the flat blank for the implant (10; 20; 30; 40; 50; 60) is between 0.2 mm and 1.2 mm, preferably approximately 0.8 mm, and the maximum longitudinal dimension is between 2 mm and 7 mm, preferably approximately 5 mm.

11. An implant according to any one of the preceding claims, **characterised in that** the triangular angle (φ) at the triangular vertex (S) of the triangular base body (11; 21; 31; 41; 51; 61) is between 60° and 160°, preferably between 100° and 120°.

12. An implant according to one of the preceding claims, **characterised in that** the implant (10; 20; 30; 40; 50; 60) has a biocompatible coating.

13. An implant according to any one of claims 1 to 12, **characterised in that** the implant (10; 20; 30; 40; 50; 60) is constructed from sheet metal, in particular from pure titanium or a titanium alloy, or from a material with a shape memory effect and/or superelastic properties, preferably from nitinol.

14. An implant according to any one of claims 1 to 12, **characterised in that** the implant (10; 20; 30; 40; 50; 60) is made wholly or partly from a plastic sheet, in particular from a polymer material, preferably from a material which has a flexibility similar to that of the human nostrils but possesses sufficient rigidity to effect a permanent and stable spreading of a nostril away from the septum.

## Revendications

1. Implant (10 ; 20 ; 30 ; 40 ; 50 ; 60) destiné à écarter au moins une aile nasale, pouvant être fixé dans le nez humain, qui, à l'état implanté, repose sur toute sa surface contre au moins une face interne d'une aile nasale et qui est constitué d'une découpe de tôle mince pourvue d'une multitude de perforations traversantes (15) ; la découpe, initialement plate, étant courbée avant l'implantation pour prendre une forme tridimensionnelle s'écartant du plan de la tôle, qui épouse la courbure naturelle de la face interne de la paroi latérale molle du nez ; l'implant (10 ; 20 ; 30 ; 40 ; 50 ; 60) repose, à l'état implanté, sur toute la surface d'une seule face interne de l'une des deux ailes nasales ; l'implant (10 ; 20 ; 30 ; 40 ; 50 ; 60) comportant un corps de base triangulaire (11 ; 21 ; 31 ; 41 ; 51 ; 61), dans lequel, à partir d'une pointe (S) de la triangle, deux arêtes (12', 13' ; 22', 23' ; 32', 33' ; 42', 43' ; 52', 53' ; 62', 63') sont disposées l'une par rapport à l'autre selon un angle triangulaire (φ), s'éloignent chacune de la pointe (S) de la triangle et s'étendent de part et d'autre respectivement en direction d'un bord d'hypoténuse (14' ; 24' ; 34' ; 44' ; 54' ; 64'); et les pointes de base du corps de base triangulaire (41 ; 51 ; 61) étant tels que, à chaque fois, une arête cathète (42' ; 52', 53' ; 62', 63') rejoint l'arête d'hypoténuse (44' ; 54' ; 64') opposée à la pointe (S) de la triangle, sont raccourcies ou tronquées, de sorte que l'extrémité de chaque arête cathète (42' ; 52', 53' ; 62', 63') est reliée, par l'intermédiaire d'une autre ligne d'arête (42x' ; 52x', 53x' ; 62x', 63x'), à une extrémité de l'arête d'hypoténuse (44' ; 54' ; 64'),
**caractérisé en ce**
**que** le corps de base triangulaire (21 ; 31 ; 41) est relié, sur l'une ou les deux arêtes de l'hypoténuse (22', 23' ; 32', 33' ; 43') par deux segments latéraux (22a, 23a ; 32a, 33a ; 43a) s'étendant chacun à partir de la pointe du triangle (S) au-delà de l'arête d'hypoténuse (24' ; 34' ; 44'), les arêtes extérieures (22a', 23a' ; 32a', 33a' ; 43a') des deux segments latéraux (22a, 23a ; 32a, 33a ; 43a) s'alignent respectivement avec leur arête cathète respective (22', 23' ; 32', 33' ; 43').

2. Implant selon la revendication 1, **caractérisé en ce qu'**au moins l'un des deux bords du cathéter (53' ; 63') et/ou le bord de l'hypoténuse (54' ; 64') opposé à la pointe du triangle (S) ne suit pas une ligne droite, mais une courbe.

3. Implant selon l'une des revendications précédentes, **caractérisé en ce que** la pointe (S) du triangle ainsi que, de préférence, tous les autres angles de l'implant (10 ; 20 ; 30 ; 40 ; 50 ; 60), au moins ceux situés à l'extérieur, sont arrondis.

4. Implant selon l'une des revendications 1 à 3, **caractérisé en ce que** l'implant (10 ; 20) est construit symétriquement par rapport à un axe de hauteur s'étendant perpendiculairement depuis la pointe du triangle (S) jusqu'à l'arête de l'hypoténuse (14' ; 24'), qui divise latéralement l'implant (10 ; 20) en deux moitiés symétriques.

5. Implant selon l'une des revendications 1 à 3, **caractérisé en ce que** l'implant (30 ; 40 ; 50 ; 60) est de structure asymétrique, un premier bord de cathéter (32' ; 42' ; 52' ; 62') présente une longueur plus grande à partir de la pointe (S) du corps de base triangulaire (31 ; 41 ; 51 ; 61) que la deuxième arête de la base (33' ; 43' ; 53' ; 63').

6. Implant selon l'une des revendications précédentes, **caractérisé en ce qu'**une ou plusieurs picots sont intégrées dans le corps de base triangulaire (11 ; 21 ; 31 ; 41 ; 51 ; 61).

7. Implant selon l'une des revendications précédentes, **caractérisé en ce qu'**au moins une partie des perforations (15) est réalisée sous forme de trous circulaires, de trous oblongs, de fentes allongées, de formes polyédriques ou en nid d'abeilles.

8. Implant selon l'une des revendications précédentes, **caractérisé en ce que** les perforations (15) présentent ensemble une surface totale supérieure à celle des parties solides (15') de l'implant (10 ; 20 ; 30 ; 40 ; 50 ; 60) qui entourent les perforations (15) et qui sont notamment en forme de nervures.

9. Implant selon l'une des revendications précédentes, **caractérisé en ce que** toutes les arêtes (12', 13', 14' ; 22', 23', 24', 22a', 23a' ; 32', 33', 34', 32a', 33a' ; 42', 43', 44', 42x', 43a' ; 52', 53', 54', 52x', 53x' ; 62', 63', 64', 62x', 63x') de l'implant (10 ; 20 ; 30 ; 40 ; 50 ; 60), au moins tous les bords extérieurs, sont arrondis dans le plan de la tôle de la découpe.

10. Implant selon l'une des revendications précédentes, **caractérisé en ce que** l'épaisseur de la tôle de la découpe plate destinée à l'implant (10 ; 20 ; 30 ; 40 ; 50 ; 60) est comprise entre 0,2 mm et 1,2 mm, de préférence d'environ 0,8 mm, et que la dimension longitudinale maximale est comprise entre 2 mm et 7 mm, de préférence d'environ 5 mm.

11. Implant selon l'une des revendications précédentes, **caractérisé en ce que** l'angle du triangle (φ) à la pointe (S) du corps de base triangulaire (11 ; 21 ; 31 ; 41 ; 51 ; 61) est compris entre 60° et 160°, de préférence entre 100° et 120°.

12. Implant selon l'une des revendications précédentes, **caractérisé en ce que** l'implant (10 ; 20 ; 30 ; 40 ; 50 ; 60) comporte un revêtement biocompatible.

13. Implant selon l'une des revendications 1 à 12, **caractérisé en ce que** l'implant (10 ; 20 ; 30 ; 40 ; 50 ; 60) est constitué d'une tôle métallique, en particulier de titane pur ou d'un alliage de titane, ou d'un matériau présentant un effet mémoire et/ou des propriétés superélastiques, de préférence du nitinol.

14. Implant selon l'une des revendications 1 à 12, **caractérisé en ce que** l'implant (10 ; 20 ; 30 ; 40 ; 50 ; 60) est fabriqué entièrement ou partiellement à partir d'une feuille de matière plastique, notamment d'un matériau polymère, de préférence d'un matériau présentant une flexibilité similaire à celle des ailes du nez humain, mais disposant d'une rigidité suffisante pour provoquer un écartement durable et stable d'une aile du nez par rapport à la cloison nasale.
